# EUROPEAN PATENT APPLICATION

(11) **EP 4 437 981 A1**
(43) Date of publication of application: **02.10.2024**
(21) Application number: 23165305.6
(22) Date of filing: 30.03.2023
(51) Int. Cl.: A61B 17/32, A61B 17/3205, A61B 18/24, A61B 17/00, A61B 18/00

(54) **CONCENTRIC SHEATH ASSEMBLY FOR AORTIC VALVE LEAFLET REMOVAL AND ASSOCIATED DEVICES, SYSTEMS, AND METHODS**

(71) Applicant: Koninklijke Philips N.V., 5656 AG Eindhoven (NL)
(72) Inventor: MORRILL, Meghan B., Eindhoven (NL)
(74) Representative: Philips Intellectual Property & Standards

(57) **Abstract**

A system includes an outer sheath and an inner sheath. The outer sheath can be positioned within a heart of a patient. The outer sheath includes a proximal portion, a distal portion, a lumen, a cutting tip positioned at the distal portion. The inner sheath is positioned within the lumen of the outer sheath. The outer sheath and the inner sheath can receive a leaflet of an aortic valve within a gap between an outer portion of the inner sheath and an inner portion of the outer sheath. The cutting tip of the outer sheath can cut the leaflet while the leaflet is received within the gap such that a cut portion of the leaflet is captured within the gap for removal from the patient.

## Description

### TECHNICAL FIELD

The subject matter described herein relates to cardiac valve replacement and, in particular, to the reducing or eliminating blood flow obstruction to the human heart during and after cardiac valve replacement. For example, a concentric sheath assembly may be utilized to remove one or more portions of leaflets of a stenosed and/or otherwise degenerated natural aortic valve or a stenosed and/or otherwise degenerated replacement aortic valve that may obstruct blood flow to the human heart during an aortic valve replacement procedure.

### BACKGROUND

One common type of valve disease is aortic valve stenosis and, with an advent of minimally invasive procedures, valve replacement is most common therapy. Transvenous/transcatheter aortic valve replacement (TAVR) is becoming more common of a procedure as technology and doctor skillsets improve. This minimally invasive approach to valve replacement is an alternative to open heart surgical aortic valve replacement (SAVR). One difference between a TAVR procedure and a SAVR procedure is, in the SAVR procedure, the natural aortic valve is removed during an open-heart procedure that is performed once, i.e. when the natural aortic valve is replaced. In a TAVR procedure, the damaged valve, whether the valve is a natural aortic valve or a previous SAVR or TAVR valve, is left in place. These valves may have anatomical abnormalities, calcification, or infection. Inserting a new valve over the previous valve may cause complications in the TAVR procedure, including valve migration, valve embolization, paravalvular leakage, patient-prosthesis mismatch, and blockage of the coronary arteries restricting blood flow to the heart.

TAVR has been approved for low-risk patients, which in general are of younger age and live longer. Recent studies have shown that the life of a TAVR valve will only be on average 8 years, so there will be an increase in replacement TAVR procedures. Old TAVR leaflets may be fibrosed, calcified, or thickened during its life creating a barrier to replacement valve. The old leaflets also pose a more serious risk of coronary obstruction than native valves.

The information included in this Introduction section of the specification, including any references cited herein and any description or discussion thereof, is included for context and/or technical reference purposes only and is not to be regarded as subject matter by which the scope of the disclosure is to be bound or otherwise limited in any manner.

### SUMMARY

Disclosed are intraluminal devices, systems, and methods that assist in the removal of old valve leaflets, whether natural aortic leaflets or previous TAVR leaflets, and prepare site for valve replacement during a cardiac valve replacement. In some instances, deployment of a new TAVR valve on top of an existing damaged natural aortic valve or previous TAVR valve may lead to complications in the cardiac valve replacement procedure. Removing old valve leaflets helps prepare the implant site for a cleaner valve deployment and operation. Removal of the leaflets may introduce issues with aortic regurgitation or aortic insufficiency in the patient. This is especially important in valve-in-valve TAVR procedure as the risk for coronary obstruction is higher. During the implantation of a replacement valve in a TAVR procedure, the action of expanding the replacement valve may push the current leaflets to the sides of the aorta and cover the openings to the coronary arteries, reducing oxygenated blood to the heart. This is a critical risk during the valve replacement, so by creating a concentric sheath and either a mechanical or laser assembly device, parts of the aortic valve leaflets may be removed prior to expanding a replacement valve.

In some aspects, in order to remove parts of the aortic valve leaflets prior to expanding a replacement valve, a set of sheaths, i.e. an inner sheath and an outer sheath, are utilized to capture one or more leaflets within a gap between the outer surface of an inner sheath and an inner portion of an outer sheath. A cutting feature on the outer sheath is then utilized to cut the one or more leaflets while the one or more leaflets are received within the gap such that a cut portion of the leaflet is captured within the gap for removal from the patient.

In an exemplary aspect, a system is provided. The system includes an outer sheath configured to be positioned within a heart of a patient, wherein the outer sheath comprises a proximal portion, a distal portion, a lumen, a cutting tip positioned at the distal portion, and an inner diameter; and an inner sheath configured to be positioned within the lumen of the outer sheath, wherein the inner sheath comprises an outer diameter, wherein the outer sheath and the inner sheath are configured to receive a leaflet of an aortic valve within a gap between the outer diameter of the inner sheath and the inner diameter of the outer sheath, wherein the cutting tip of the outer sheath is configured to cut the leaflet while the leaflet is received within the gap such that a cut portion of the leaflet is captured within the gap for removal from the patient. The inner diameter of the outer sheath defines an inner surface of at least a portion of outer sheath, or with other words, the inner diameter of the outer sheath defines an inner portion of the outer sheath. The outer diameter of the inner sheath defines an outer surface of at least a portion of inner sheath, or with other words, the outer diameter of the inner sheath defines an outer portion of the inner sheath.

In some aspects, the inner sheath comprises an outer surface with a hydrophilic coating. In some aspects, the inner sheath comprises a proximal portion and a distal portion, and at least one of the proximal portion of the inner sheath or the proximal portion of the outer sheath comprises a hemostasis valve. In some aspects, the inner sheath comprises a proximal portion and a distal portion, and at least one of the distal portion of the inner sheath or the distal portion of the outer sheath comprises a radiopaque marker. In some aspects, the inner sheath comprises a proximal portion and a distal portion, and at least one of the proximal portion of the inner sheath or the proximal portion of the outer sheath comprises a visible marker. In some aspects, the cutting tip comprises at least one of a sharp edge or an optical fiber. In some aspects, the system includes a pump configured to provide suction within the gap such that the cut portion of the leaflet is captured within the gap. In some aspects, the inner sheath comprises an outer surface with a screw thread, and the inner sheath is configured to be rotated relative to the outer sheath such that the cut portion of the leaflet is captured within the gap by the screw thread. In some aspects, the system includes a motor configured to rotate the inner sheath. In some aspects, the outer sheath comprises an inner surface, the inner sheath comprises an outer surface, at least one of the inner surface of the outer sheath or the outer surface of the inner sheath comprises a frictional material configured to capture the cut portion of the leaflet within the gap. In some aspects, the system includes a valve delivery catheter configured to be positioned within the lumen of the outer sheath, the valve delivery catheter is movable relative to the outer sheath, and the valve delivery catheter comprises a transcatheter aortic valve replacement (TAVR) valve removably coupled at a distal portion of the valve delivery catheter.

In an exemplary aspect, a method is provided. The method includes moving an inner sheath within an aorta and through an aortic valve such that a distal portion of the inner sheath is positioned within a left ventricle of a heart; moving an outer sheath within the aorta and along the inner sheath such the inner sheath is positioned within a lumen of the outer sheath, wherein the outer sheath comprises a distal portion and a cutting tip positioned at the distal portion of the outer sheath; receiving a leaflet of the aortic valve within a gap between an outer diameter of the inner sheath and an inner diameter of the outer sheath; cutting, with the cutting tip, the leaflet while the leaflet is received within the gap; capturing a cut portion of the leaflet within the gap; and removing the cut portion of the leaflet from a body of a patient.

In some aspects, the distal portion of the inner sheath is positioned within the left ventricle such that: the leaflet contacts the inner sheath during diastole to prevent mitral regurgitation; and the leaflet is spaced from the inner sheath during systole to allow blood flow from the left ventricle to the aorta. In some aspects, cutting the leaflet comprises moving the distal portion of the outer sheath distally towards the distal portion of the inner sheath such that the distal portion of the outer sheath is positioned within the left ventricle. In some aspects, the distal portion of the outer sheath is positioned within the left ventricle such that: the leaflet contacts the outer sheath during diastole to prevent mitral regurgitation; and the leaflet is spaced from the outer sheath during systole to allow blood flow from the left ventricle to the aorta. In some aspects, capturing the cut portion of the leaflet comprises: providing suction within the gap. In some aspects, the inner sheath comprises an outer surface with a screw thread, and capturing the cut portion of the leaflet comprises rotating the inner sheath relative to the outer sheath to capture the cut portion of the leaflet with the screw thread. In some aspects, at least one of an inner surface of the outer sheath or an outer surface of the inner sheath comprises a frictional material, and capturing the cut portion of the leaflet comprises contacting the cut portion within the frictional material. In some aspects, the method includes moving a valve delivery catheter through the lumen of the outer sheath, and the valve delivery catheter comprises a transcatheter aortic valve replacement (TAVR) valve removably coupled at a distal portion of the valve delivery catheter.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of aspects of the present disclosure, e.g., as defined in the claims, is provided in the following written description of various examples and/or aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1A illustrates a side view of a human heart according to aspects of the present disclosure.
Fig. 1B is a cross-sectional side view of a human heart according to aspects of the present disclosure.
Fig. 2 is a cross-sectional side view of an aortic valve replacement in a human heart according to aspects of the present disclosure.
Fig. 3A is a diagrammatic cross-sectional top view of an aortic valve replacement of a degenerated natural aortic valve utilizing a TAVR valve, according to aspects of the present disclosure.
Fig. 3B is a diagrammatic cross-sectional top view of a valve-in-valve replacement of a degenerated TAVR valve utilizing a new TAVR valve, according to aspects of the present disclosure.
Fig. 3C is a diagrammatic cross-sectional top view of a valve-in-valve replacement of a degenerated surgical aortic valve replacement (SAVR) valve utilizing a new TAVR valve, according to aspects of the present disclosure.
Fig. 4A is a diagrammatic top view of an aortic valve, according to aspects of the present disclosure.
Fig. 4B is a diagrammatic cross-sectional side view of a TAVR implant in an aortic valve according to aspects of the present disclosure.
Fig. 5 is diagrammatic, schematic view of a system according to aspects of the present disclosure.
Fig. 6 is a schematic diagram of a processing system according to aspects of the present disclosure.
Fig. 7 is a diagrammatic cross-sectional top-view of an intraluminal device according to aspects of the present disclosure.
Fig. 8 is a diagrammatic cross-sectional top-view of an inner sheath according to aspects of the present disclosure.
Fig. 9 is a diagrammatic cross-sectional top-view of an outer/cutting sheath according to aspects of the present disclosure.
Fig. 10 is a diagrammatic cross-sectional top-view of an outer/cutting sheath according to aspects of the present disclosure.
Fig. 11 is a diagrammatic view of an intraluminal device according to aspects of the present disclosure.
Figs. 12A and 12B are diagrammatic cross-sectional side views of an aortic valve according to aspects of the present disclosure.
Fig. 13 is diagrammatic view of an intraluminal device inserted into an aortic valve of a human heart according to aspects of the present disclosure.
Fig. 14 is a diagrammatic cross-sectional side view of an aortic valve according to aspects of the present disclosure.
Fig. 15 is diagrammatic view of an intraluminal device inserted into an aortic valve of a human heart according to aspects of the present disclosure.
Fig. 16 is diagrammatic view of an intraluminal device inserted into an aortic valve of a human heart according to aspects of the present disclosure.
Fig. 17A is a diagrammatic cross-sectional top-view of an intraluminal device according to aspects of the present disclosure.
Fig. 17B illustrates a cross-sectional view of the intraluminal device as seen along the lines of the section A-A of Fig. 17A taken therein, according to aspects of the present disclosure.
Fig. 18 is a diagrammatic cross-sectional top-view of an intraluminal device according to aspects of the present disclosure.
Fig. 19 is a diagrammatic cross-sectional top-view of an intraluminal device according to aspects of the present disclosure.
Figs. 20A and 20B are diagrammatic cross-sectional side views of an aortic valve according to aspects of the present disclosure.
Fig. 21 is a diagrammatic cross-sectional side view of an aortic valve according to aspects of the present disclosure.
Fig. 22 is a diagrammatic cross-sectional side view of an aortic valve according to aspects of the present disclosure.
Fig. 23 is a flow diagram of the process performed in removing one or more portions of leaflets of a degenerated natural aortic valve or a degenerated replacement aortic valve according to aspects of the present disclosure.

### DETAILED DESCRIPTION

For the purposes of promoting an understanding of the principles of the present disclosure, reference will now be made to the examples illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one example and/or aspect may be combined with the features, components, and/or steps described with respect to other examples and/or aspects of the present disclosure. Additionally, while the description below may refer to blood vessels, it will be understood that the present disclosure is not limited to such applications. For example, the devices, systems, and methods described herein may be used in any body chamber or body lumen, including an esophagus, veins, arteries, intestines, ventricles, atria, or any other body lumen and/or chamber. For the sake of brevity, however, the numerous iterations of these combinations will not be described separately.

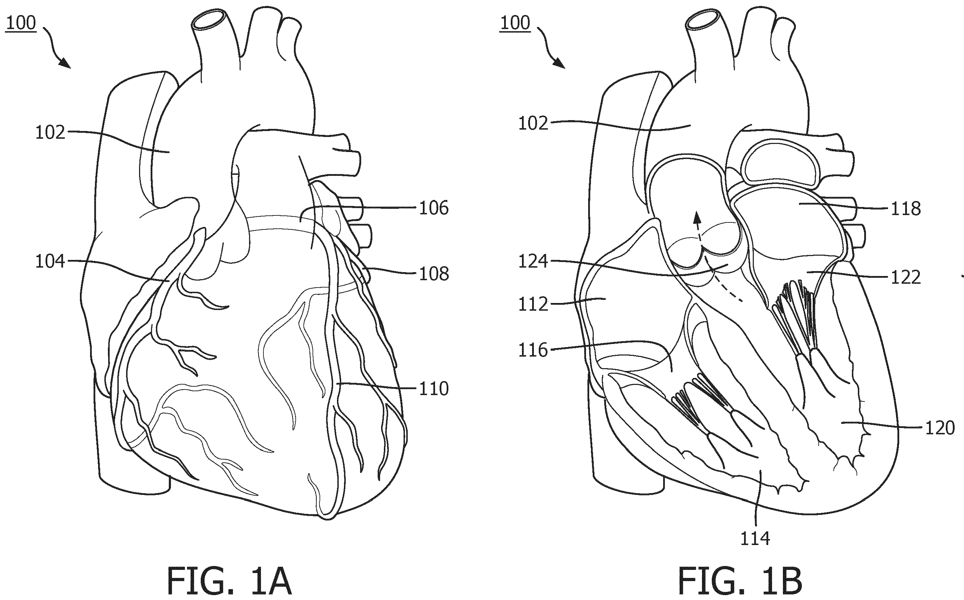

Referring to Fig. 1A, shown is a side view of a human heart 100 according to aspects of the present disclosure. Visible are an aorta 102 from which stems a right coronary artery 104 and a left main coronary artery 106. The left main coronary artery 106 branches into a left circumflex coronary artery 108 and a left anterior descending coronary artery 110. The right coronary artery 104, the left main coronary artery 106, the left circumflex coronary artery 108, and a left anterior descending coronary artery 110 are the arteries that provide oxygen-rich blood to muscles of the human heart 100.

Fig. 1B is a cross-sectional side view of a human heart 100 according to aspects of the present disclosure. Visible are a right atrium 112 and a right ventricle 114. In that regard, oxygen-poor blood enters the human heart 100 in the right atrium 112 and travels to the right ventricle 114 through the tricuspid valve 116. The oxygen-poor blood leaves the right ventricle 114 and travels to the lungs. Also visible are a left atrium 118 and a left ventricle 120. In that regard, oxygen-rich blood is received from the lungs in the left atrium 118 and travels to the left ventricle 120 through the mitral valve 122. The oxygen-rich blood leaves the left ventricle 120 and goes out to the body through the aorta 102 via an aortic valve 124.

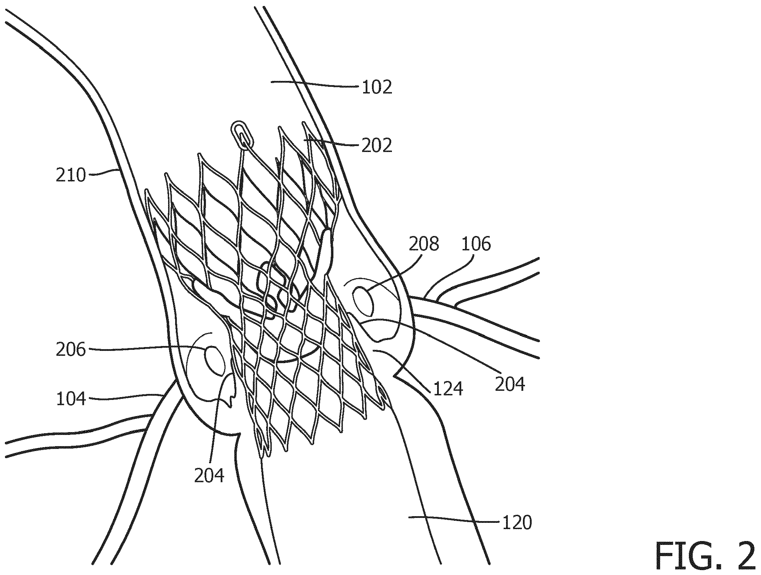

Fig. 2 is a cross-sectional side view of an aortic valve 124 replacement in a human heart 100 according to aspects of the present disclosure. In some aspects, e.g., when aortic valve stenosis has occurred to the aortic valve 124 that keeps blood flowing in the correct direction from the left ventricle 120 to the aorta 102, a transvenous/transcatheter aortic valve repair (TAVR) procedure may be performed to replace the natural aortic valve 124 with a replacement aortic valve 202. In some instances, portions of one or more leaflets 204 of the natural aortic valve 124 may be resected so that openings 206 and 208 to the right coronary artery 104 and the left main coronary artery 106 remain unobstructed so that oxygen-rich blood may flow to the muscles of the human heart, when the one or more leaflets 204 are pressed against the natural heart wall 210. The natural heart wall 210 may be a natural aorta wall, a natural heart chamber wall, or a natural aortic valve wall.

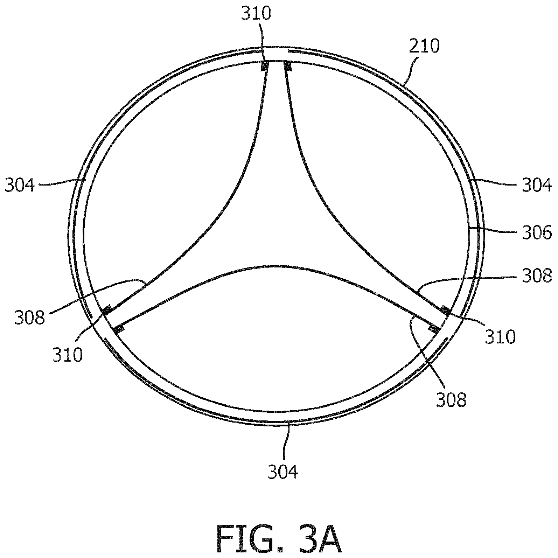

Fig. 3A is a diagrammatic cross-sectional top view of an aortic valve replacement of a degenerated natural aortic valve utilizing a TAVR valve, according to aspects of the present disclosure. Visible are the natural heart wall 210, the natural aortic valve leaflets 304, the TAVR valve wall 306, the TAVR valve leaflets 308, and the TAVR commissural tabs 310. The TAVR valve leaflets 308 are constructed from bovine animal tissue that are coupled to the wire frame of the TAVR valve wall 306 via the commissural tabs 310. When inserted, the TAVR valve pushes the remaining unresected portions of the natural aortic valve leaflets 304 against the natural heart wall 210 such that the natural aortic valve leaflets 304 are pinned and/or secured between an outside of the TAVR valve wall 306 and the natural heart wall 210.

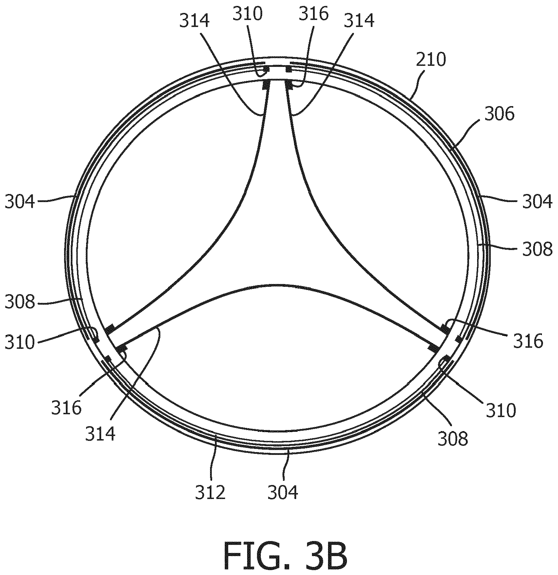

Fig. 3B is a diagrammatic cross-sectional top view of a valve-in-valve replacement of a degenerated TAVR valve utilizing a new TAVR valve, according to aspects of the present disclosure. Visible are the natural heart 210, the natural aortic valve leaflets 304, the degenerated TAVR valve wall 306, the degenerated TAVR valve leaflets 308, the degenerated TAVR commissural tabs 310, the new TAVR valve wall 312, the new TAVR valve leaflets 314, and the new TAVR commissural tabs 316. The new TAVR valve leaflets 314 are constructed from bovine animal tissue that are coupled to the wire frame of the new TAVR valve wall 312 via the commissural tabs 316. When inserted, the TAVR valve pushes the remaining unresected portions of the degenerated TAVR valve leaflets 308 against an inside of the degenerated TAVR valve wall 306 such that the degenerated TAVR valve leaflets 308 are pinned and/or secured between an outside of the new TAVR valve wall 312 and the inside wall of the degenerated TAVR valve wall 306.

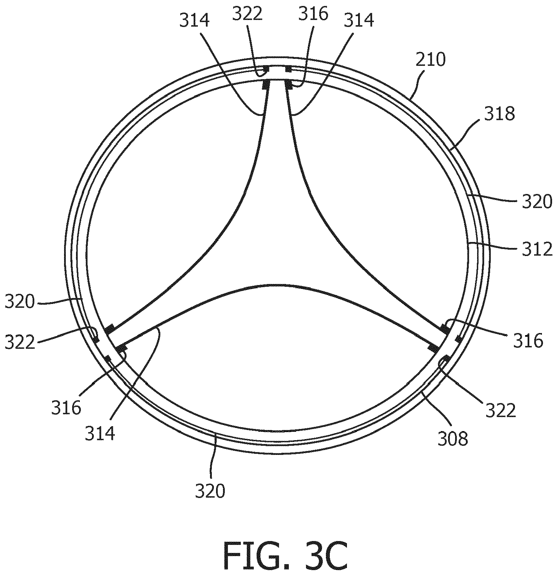

Fig. 3C is a diagrammatic cross-sectional top view of a valve-in-valve replacement of a degenerated surgical aortic valve replacement (SAVR) valve utilizing a new TAVR valve, according to aspects of the present disclosure. Visible are the natural heart wall 210, a degenerated SAVR valve wall 318, the degenerated SAVR valve leaflets 320, the degenerated SAVR commissural tabs 322, the new TAVR valve wall 312, the new TAVR valve leaflets 314, and the new TAVR commissural tabs 316. The new TAVR valve leaflets 314 are constructed from bovine animal tissue that are coupled to the wire frame of the new TAVR valve wall 312 via the commissural tabs 316. When inserted, the TAVR valve pushes the remaining unresected portions of the degenerated SAVR valve leaflets 320 against an inside of the degenerated SAVR valve wall 318 such that the degenerated TAVR valve leaflets 320 are pinned and/or secured between an outside of the new TAVR valve wall 312 and the inside wall of the degenerated SAVR valve wall 318.

The natural valve, TAVR valve, and/or SAVR valve can be degenerated (e.g., does not function as it should) as a result of various causes. For example, the natural valve, TAVR valve, and/or SAVR valve can be degenerated because it is stenosed, because it is regurgitant, etc.

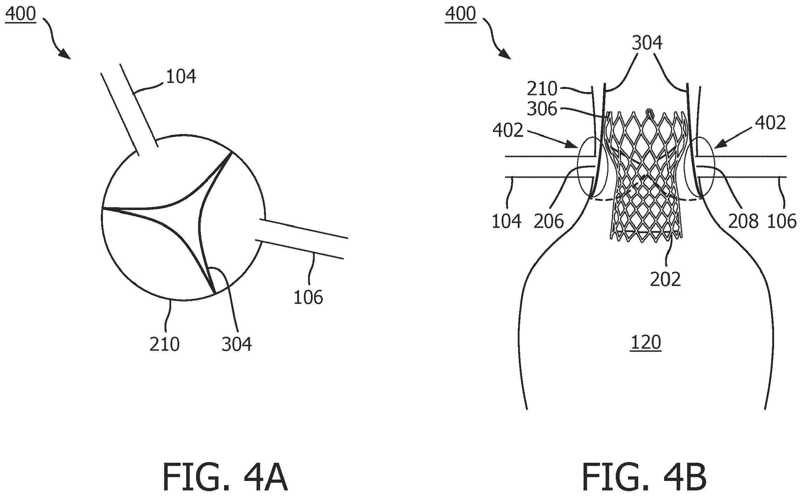

Fig. 4A is a diagrammatic top view of an aortic valve 400, according to aspects of the present disclosure. Visible are the natural heart wall 210, the aortic valve leaflets 304, the right coronary artery 104, and the left main coronary artery 106. Fig. 4B is a diagrammatic cross-sectional side view of a TAVR implant in an aortic valve 400 according to aspects of the present disclosure. Visible are the natural heart wall 210, the aortic valve leaflets 304, the right coronary artery 104, and the left main coronary artery 106. In some aspects, when a replacement aortic valve 202 is implanted and the aortic valve leaflets 304 are not resected or insufficiently resected, the aortic valve leaflets 304, when pinned and/or secured between an outside of the TAVR valve wall 306 and the natural heart wall 210, obstruct the openings 206 and 208 to the right coronary artery 104 and the left main coronary artery 106, respectively, as indicated by areas 402.

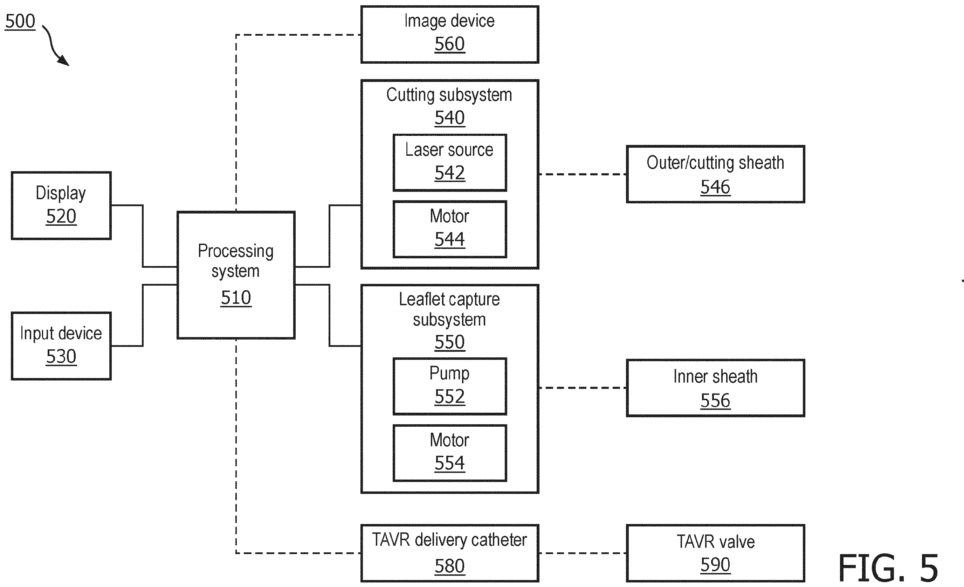

Fig. 5 is diagrammatic, schematic view of a system 500 according to aspects of the present disclosure. The system 500 may be configured to evaluate (e.g., assess), display, and/or control (e.g., modify) one or more aspects of a cardiac valve replacement. For instance, the system 500 may be utilized to monitor and/or control one or more portions of a cardiac valve replacement process while reducing and/or eliminating aortic regurgitation, as described in greater detail below. In this regard, the system 500 may be used to assess coronary vessels and/or heart tissue (e.g., the myocardium). As illustrated, the system 500 may include a processing system 510 in communication with a display device 520 (e.g., an electronic display or monitor), an input device 530 (e.g., a user input device, such as a keyboard, mouse, joystick, microphone, and/or other controller or input device), a cutting subsystem 540, a leaflet capture subsystem 550, and/or an imaging device 560. As illustrated, the system 500 may further include a TAVR delivery catheter 580 and a TAVR valve 590, which may be completely mechanical or connected to processing system 510 in order to provide intraluminal data to processing system 510.

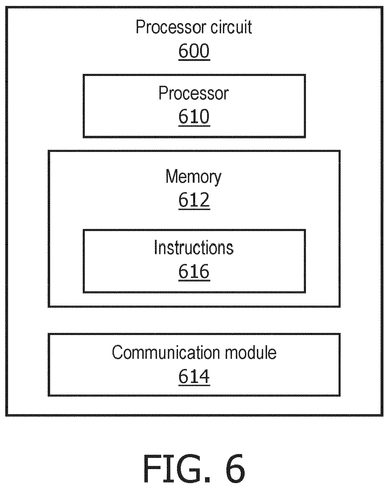

The processing system 510 is generally representative of any device suitable for performing the processing and analysis techniques disclosed herein. In some aspects, the processing system 510 includes a processor circuit, such as the processor circuit 600 of Fig. 6. In some aspects, the processing system 510 is programmed to execute steps associated with the data acquisition, analysis, and/or instrument (e.g., device) control described herein. Accordingly, it is understood that any steps related to data acquisition, data processing, instrument control, and/or other processing or control aspects of the present disclosure may be implemented by the processing system 510 (e.g., computing device) using corresponding instructions stored on or in a non-transitory computer readable medium accessible by the computing device. In some instances, the processing system 510 is a console device. Further, it is understood that in some instances the processing system 510 includes one or a plurality of computing devices, such as computers, with one or a plurality of processor circuits. In this regard, it is particularly understood that the different processing and/or control aspects of the present disclosure may be implemented separately or within predefined groupings using a plurality of computing devices. Any divisions and/or combinations of the processing and/or control aspects described below across multiple computing devices are within the scope of the present disclosure.

Fig. 6 is a schematic diagram of a processing system according to aspects of the present disclosure. The processor circuit 600 may be implemented in and/or as part of the processing system 510 of Fig. 5. As shown, the processor circuit 600 may include a processor 610, a memory 612, and a communication module 614. These elements may be in direct or indirect communication with each other, for example via one or more buses.

The processor 610 may include a central processing unit (CPU), a digital signal processor (DSP), an ASIC, a controller, an FPGA, another hardware device, a firmware device, or any combination thereof configured to perform the operations described herein. The processor 610 may also be implemented as a combination of computing devices, e.g., a combination of a DSP and a microprocessor, a plurality of microprocessors, one or more microprocessors in conjunction with a DSP core, or any other such configuration.

The memory 612 may include a cache memory (e.g., a cache memory of the processor 610), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In some instances, the memory 612 includes a non-transitory computer-readable medium. The memory 612 may store instructions 616. The instructions 616 may include instructions that, when executed by the processor 610, cause the processor 610 to perform the operations described herein with reference to the processing system 510 (Fig. 5). Instructions 616 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 614 may include any electronic circuitry and/or logic circuitry to facilitate direct or indirect communication of data between various components of the processor circuit 600 and/or the processing system 510 (Fig. 5). Additionally or alternatively, the communication module 614 may facilitate communication of data between the processor circuit 600, the display device 520, the input device 530, the cutting subsystem 540, the leaflet capture subsystem 550, the imaging device 560, the TAVR delivery catheter 580, and/or the like. In this regard, the communication module 614 may be an input/output (I/O) device interface, which may facilitate communicative coupling between the processor circuit 600 and (I/O) devices, such as the input device 530. Moreover, the communication module 614 may facilitate wireless and/or wired communication between various elements of the processor circuit 600 and/or the devices and systems of the system 500 using any suitable communication technology, such as a cable interface such as a USB, micro-USB, Lightning, or FireWire interface, Bluetooth, Wi-Fi, ZigBee, Li-Fi, or cellular data connections such as 2G/GSM, 3G/UMTS, 4G/LTE/WiMax, or 5G.

Turning back now to Fig. 5, the imaging device 560 may include an x-ray system, angiography system, fluoroscopy system, ultrasound system, computed tomography (CT) system, a magnetic resonance imaging (MRI) system, other suitable imaging devices, and/or combinations thereof. The imaging device 650 may additionally or alternatively include a nuclear medicine imaging device, such as a gamma camera or a single-photon emission computed tomography (SPECT) system, other suitable devices, and/or combinations thereof. In some aspects, the imaging device 560 may be configured to acquire imaging data of anatomy, such as the heart and blood vessels, while the imaging device 560 is positioned outside of the body of the patient. The imaging data may be visualized in the form of two-dimensional and/or three-dimensional images of the heart, blood vessel, and/or other anatomy. In some aspects, the imaging device 560 may be an internal device that is positioned inside the patient body. For example, the imaging device 560 may be an intracardiac echocardiography (ICE) catheter that obtains images while positioned within a heart chamber. In some aspects, the imaging device 560 may be positioned outside of the particular anatomy that is being imaged (e.g., blood vessels and/or heart), but is positioned inside the patient body. For example, the imaging device 560 may be a transesophageal echocardiography (TEE) probe that obtains images while positioned within an esophagus.

Moreover, the imaging device 560 may obtain images of the heart that are indicative of the health of the cardiac muscle or myocardium. In particular, the imaging device 560 may be configured to acquire imaging data that illustrates myocardial perfusion (e.g., myocardial perfusion imaging, MPI, data). For example, MPI data may be collected by imaging a radiopharmaceutical agent, such as thallium, in the patient's heart muscle using a SPECT system. Additionally or alternatively, the imaging data may be obtained by imaging one or more radiopaque markers embedded in body of an intraluminal device, for example, the outer/cutting sheath 546, the inner sheath 556, and/or the TAVR delivery catheter 580 to enable tracking and identify the location of the intraluminal device using fluoroscopy. In any case, the imaging data may illustrate vasculature and/or muscle mass with blood flow and/or vasculature and/or muscle mass that lack of blood flow in areas of the heart.

The leaflet capture subsystem 550 may control pump 552 and/or motor 554 to capture the leaflets of the degenerated natural aortic valve or a degenerated replacement aortic valve. In some instances, pump 552 may be omitted. In that regard, suction may be provided manually (e.g., syringe at the proximal portion with plunger and barrel; pull plunger along inside of barrel to provide suction). In some instances, motor 554 may be omitted. In that regard, rotation may be provided manually (turning proximal portion of inner sheath). In some aspects, the human heart/heart cycle has two portions: systole and diastole. In that regard, the inner sheath 556 is advanced through the aortic valve during systole, when the heart muscle contracts and pumps blood from the chambers into the arteries. Then, during diastole when the heart muscle relaxes and the chambers of the heart are allowed to fill with blood, the leaflets of the aortic valve press against the outer surface of the inner sheath 556. In some instances, the leaflet capture subsystem 550 may be configured to apply negative pressure and/or suction via pump 552 between the inner sheath 556 and the outer/cutting sheath 546 such that the leaflets are suctioned into a gap between an outer surface or portion of the inner sheath 556 and an inner surface or portion of the outer/cutting sheath 546. In that regard, the suction may be for receiving the leaflet within the gap, capturing the cut portion of the leaflet within the gap, and/or removing the cut portion of the leaflet from the body. In some instances, the leaflet capture subsystem 550 may be configured to rotate via motor 554, such as an Archimedes screw or helical retraction on the outside of the inner sheath 556, which moves the leaflets between an outside of the inner sheath 556 and an inside of the outer/cutting sheath 546. In some instances, the outside of the inner sheath 556 may comprise a frictional material, texturized, and/or coated with a friction surface such that, during diastole, the leaflets of the aortic valve adhere to the outer surface of the inner sheath 556 and, as the outer/cutting sheath is advanced, positions the leaflets between an outside of the inner sheath 556 and an inside of the outer/cutting sheath 546.

The cutting subsystem 540 may control laser source 542 and/or motor 544 for controlling a laser cutter and/or cutting tip disposed on a distal end of the outer/cutting sheath 546. In that regard, once the leaflets of the degenerated natural aortic valve or a degenerated replacement aortic valve are positioned between the outside of the inner sheath 556 and the inside of the outer/cutting sheath 546, the outer/cutting sheath 546 may be advanced. In some aspects, the cutting subsystem 540 is configured to enable the laser source 542, so that one or more optical fibers disposed on a distal end of the outer/cutting sheath 546 may cut and/or resect the degenerated leaflets of the natural aortic valve or the replacement aortic valve. In some aspects, the cutting subsystem 540 is configured to enable the motor 544, so that one or more mechanical blades disposed on a distal end of the outer/cutting sheath 546 may cut and/or resect the degenerated leaflets of the natural aortic valve or the replacement aortic valve. Once the leaflets of the degenerated natural aortic valve or a degenerated replacement aortic valve, TAVR delivery catheter 580 delivers the TAVR valve 590 to the aortic valve area of the human heart, such that the TAVR valve 590 replaces either the degenerated natural aortic valve or a degenerated replacement aortic valve.

The system 500 includes a display device 520 that is communicatively coupled to the processing system 510. In some aspects, the display device 520 is a component of the processing system 510, while in other aspects, the display device 520 is distinct from the processing system 510. In some aspects, the display device 520 is a monitor integrated in a console device or a standalone monitor (e.g., a flat panel or flat screen monitor). The processing system 510 may be configured to generate a visual display (e.g., screen display) based on imaging data from an imaging device. The processing system 510 may provide (e.g., output) the screen display to the display device 520. To that end, the display device 520 may be configured to output (e.g., display) a two-dimensional image and/or a two-dimensional representation of the heart, blood vessels, and/or other anatomy, which may be included in the screen display. In some aspects, the display device 520 is configured to output a three-dimensional graphical representation of the heart, blood vessels, and/or other anatomy. For instance, the display device 520 may be a holographic display device configured to output a three-dimensional holographic display of anatomy. Any suitable display device is within the scope of this disclosure, including self-contained monitors, projection/screen systems, head-up display systems, etc. The display device may implement principles based on moving reflective microelectromechanical systems (MEMS), laser plasma, electro-holography, etc. In some aspects, the display device 520 is implemented as a bedside controller having a touch-screen display as described, for example, in U.S. Provisional Application No. 62/049,265, titled "Bedside Controller for Assessment of Vessels and Associated Devices, Systems, and Methods," and filed September 11, 2014, the entirety of which is hereby incorporated by reference herein.

The system 500 includes an input device 530 that is communicatively coupled to the processing system 510. The input device 530 may be a peripheral device, such as a touch sensitive pad, a touch-screen, a joy-stick, a keyboard, mouse, trackball, a microphone, an imaging device, and/or the like. In other aspects, the user interface device is part of the display device 520, which may be a touch-screen display, for example. Moreover, a user may provide an input to the processing system 510 via the input device 530. In particular, the input device 530 may enable a user to control, via inputs to the processing system 510, one or more of the components of the system 500, such as the cutting subsystem 540, the leaflet capture subsystem 550, the imaging device 560, the TAVR delivery catheter 580, or the processing system 510 itself. Additionally or alternatively, the input device 530 may facilitate interaction with a screen display provided at the display device 520. For instance, a user may select, edit, view, or interact with portions of the screen display (e.g., a GUI) provided at the display device 520 via the input device 530.

The system 500 may include various connectors, cables, interfaces, connections, etc., to communicate between the elements of the cutting subsystem 540, the leaflet capture subsystem 550, the imaging device 560, the TAVR delivery catheter 580, the processing system 510, the display device 520, and/or the input device 530. In some aspects, for example, the communication module 614 (Fig. 6), which may be included in the processing system 510, may include such connectors, interfaces, and/or the like. In this regard, the processing system 510 may communicate and/or control one or more components of the processing system 510 via mechanical and/or electromechanical signaling and/or controls. Further, the illustrated communication pathways are exemplary in nature and should not be considered limiting in any way. In this regard, it is understood that any communication pathway between the components of system 500 may be utilized, including physical connections (including electrical, optical, and/or fluid connections), wireless connections, and/or combinations thereof. In this regard, it is understood that the one or more of the components of the system 500 may communicate via a wireless connection in some instances. In some instances, the one or more components of the system 500 and/or other systems (e.g., of a hospital or health services provider) communicate via a communication link over a network (e.g., intranet, internet, telecommunications network, and/or other network).

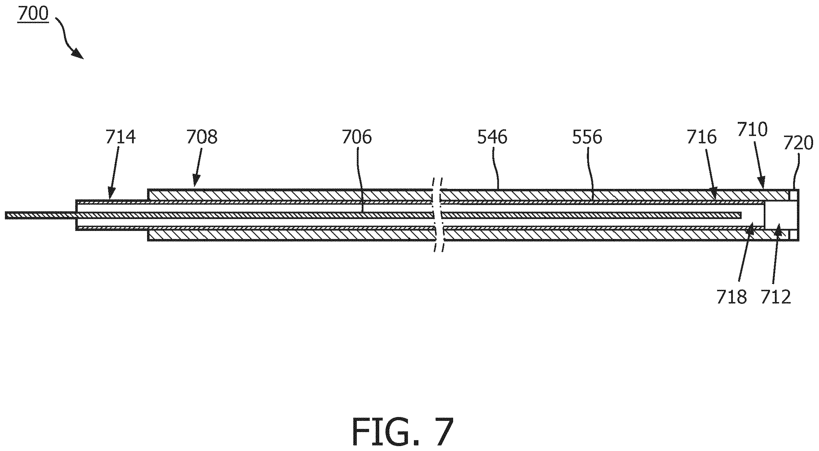

Fig. 7 is a diagrammatic cross-sectional top-view of an intraluminal device 700 according to aspects of the present disclosure. In the aspects of Fig. 7, visible are an outer/cutting sheath 546, an inner sheath 556, and guidewire 706. In some instances, the guidewire 706 is inserted through a body lumen such as an artery, e.g. the femoral artery, and advanced to the aorta 102 of the human heart 100. More specifically, the guidewire 706 is advanced to the aortic valve 124 region within the aorta 102. In some instances, the outer/cutting sheath 546 and/or the inner sheath 556 is placed over the guidewire 706 and advanced to the aortic valve 124 region. The outer/cutting sheath 546 includes a proximal end 708, a distal end 710, and a sheath lumen 712. Similarly, the inner sheath 556 includes a proximal end 714, a distal end 716, and a sheath lumen 718. In some instances, the distal end 716 of the inner sheath 556 is configured to be inserted into sheath lumen 712 at the proximal end 708 of the outer/cutting sheath 546. In that regard, the inner sheath 556 may be placed within the sheath lumen 712 of the outer/cutting sheath 546, the distal end 716 of the inner sheath 556 being placed over the guidewire 706, and the inner sheath 556 and the outer/cutting sheath 546 being placed within the body lumen and advanced together to the aortic valve 124 region. In some instances, the distal end 710 of the outer/cutting sheath 546 may be placed over the guidewire 706 in the sheath lumen 712, placed within the body lumen, and advanced to the aortic valve 124 region. In that regard, the distal end 716 of the inner sheath 556 may be inserted into sheath lumen 712 at the proximal end 708 of the outer/cutting sheath 546 and then advanced to the aortic valve 124 region. In some instances, the distal end 716 of the inner sheath 556 may be placed over the guidewire 706 in sheath lumen 718, placed within the body lumen, and advanced to the aortic valve 124 region. In that regard, the distal end 710 of the outer/cutting sheath 546 may be inserted over proximal end 714 of the inner sheath 556 and then advanced to the aortic valve 124 region. In some instances, the outer/cutting sheath 546 is a laser sheath. In some instances, the outer/cutting sheath 546 is a cutting sheath. In that regard, the outer/cutting sheath 546 includes a cutting tip 720 disposed at a distal end 710.

Aspects of the present disclosure may include features described in U.S. Provisional App. No. 63/415,724, filed October 13, 2022, and titled "Co-Registered Temporary Valve for Cardiac Valvular Interventions and Associated Devices, Systems, and Methods", and App. No. 63/443,811, filed on February 07, 2023, and titled "Sheath and Balloon Catheter Assembly for Aortic Valve Leaflet Removal and Associated Devices, Systems, And Methods", the entireties of which are hereby incorporated by reference herein.

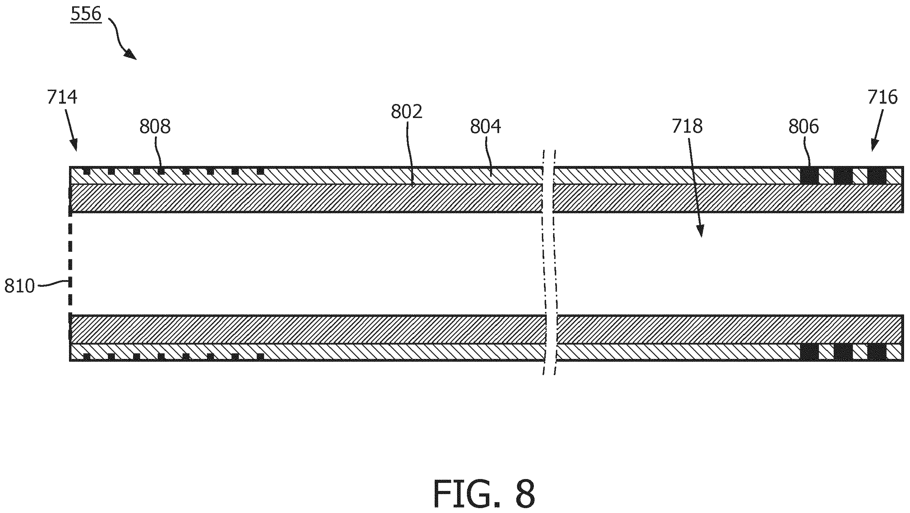

Fig. 8 is a diagrammatic cross-sectional top-view of an inner sheath 556 according to aspects of the present disclosure. In some instances, the inner sheath 556 includes a proximal end 714, a distal end 716, and a sheath lumen 718. In some instances, an outer wall 802 of the inner sheath 556 may be coated with a hydrophilic coating 804. In some instances, disposed within the hydrophilic coating 804 on the distal end 716 of the inner sheath 556 are a set of radiopaque markers 806 to enable tracking and to identify the location of the inner sheath 556 using fluoroscopy. In some instances, the set of radiopaque markers 806 may be spaced by a known distance. In some instances, disposed within the hydrophilic coating 804 on the proximal end 714 of the inner sheath 556 are a set of visible markers 808 to enable tracking and to identify a depth of the inner sheath 556 by the human operator. In that regard, the set of visible markers 808 are optically visible, i.e. visible to the bare eye, because the proximal end 714 is positioned outside of the body of patient. In some instances, the set of visible markers 808 may be spaced by known distance. In some instances, a hemostasis valve 810 may disposed on the proximal end of the sheath lumen 718. In that regard, the hemostasis valve 810 is designed to minimize fluid loss and backflow through the sheath lumen 718.

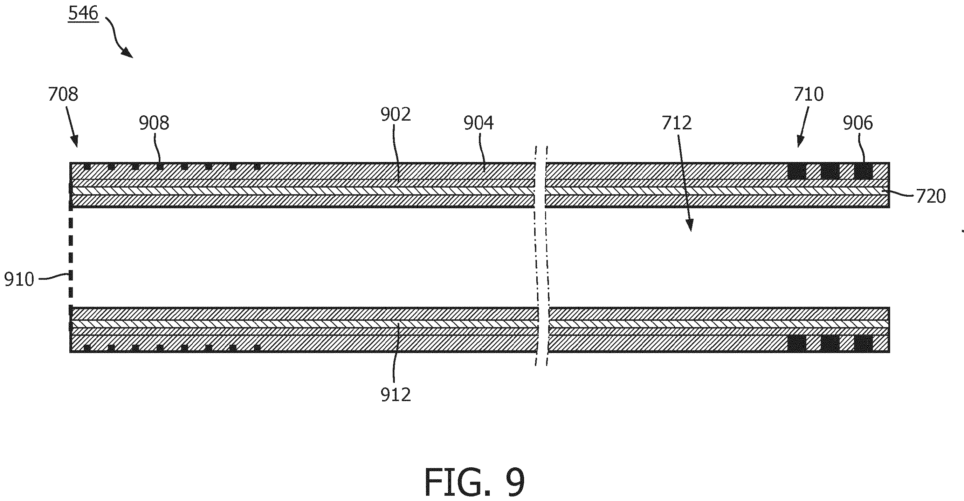

Fig. 9 is a diagrammatic cross-sectional top-view of an outer/cutting sheath 546 according to aspects of the present disclosure. In some aspects, the outer/cutting sheath 546 includes a proximal end 708, a distal end 710, and a sheath lumen 712. In some instances, an outer wall 902 of the outer/cutting sheath 546 may be coated with a hydrophilic coating 904. In some instances, disposed within the hydrophilic coating 904 on the distal end 710 of the outer/cutting sheath 546 are a set of radiopaque markers 906 to enable tracking and to identify the location of the outer/cutting sheath 546 using fluoroscopy. In some instances, the set of radiopaque markers 906 may be spaced by a known distance. In some instances, disposed within the hydrophilic coating 904 on the proximal end 708 of the outer/cutting sheath 546 are a set of visible markers 908 to enable tracking and to identify a depth of the outer/cutting sheath 546 by the human operator. In that regard, the set of visible markers 908 are optically visible, i.e. visible to the bare eye, because the proximal end 708 is positioned outside of the body of patient. In some instances, the set of visible markers 908 may be spaced by known distance. In some instances, a hemostasis valve 910 may disposed on the proximal end of the sheath lumen 712. In that regard, the hemostasis valve 910 is designed to minimize fluid loss and backflow through the sheath lumen 712. In some instances, where the outer/cutting sheath 546 is a laser sheath, one or more optical fibers 912 may traverse the length of the outer/cutting sheath 546. In that regard, a proximal end of the one or more optical fibers 912 may be coupled to a laser source (laser source 542 of Fig. 5) such that the laser source emits a light that travels along the one or more optical fibers 912 from the proximal end 708 to a distal end 710. In some aspects, the distal end of the one or more optical fibers 912 may end at the distal end 710 of the outer/cutting sheath 546 forming cutting tip 720 such that the light emitted by the one or more optical fibers 912 may cut and/or resect the degenerated leaflets of the natural aortic valve or the replacement aortic valve. Aspects of the outer/cutting sheath may include features similar to the GlideLight^{®} laser sheath available from Koninklijke Philips NV.

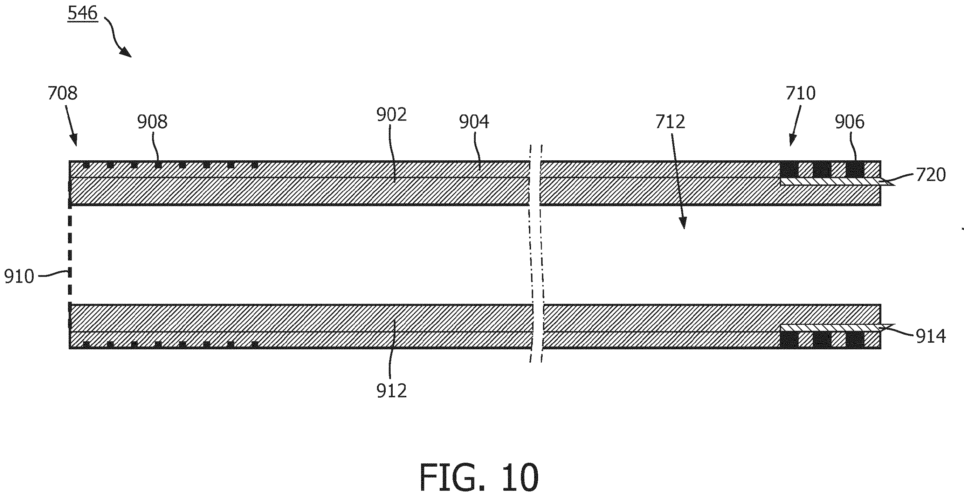

Fig. 10 is a diagrammatic cross-sectional top-view of an outer/cutting sheath 546 according to aspects of the present disclosure. In some aspects, the outer/cutting sheath 546 includes a proximal end 708, a distal end 710, and a sheath lumen 712. In some instances, an outer wall 902 of the outer/cutting sheath 546 may be coated with a hydrophilic coating 904. In some instances, disposed within the hydrophilic coating 904 on the distal end 710 of the outer/cutting sheath 546 are a set of radiopaque markers 906 to enable tracking and to identify the location of the outer/cutting sheath 546 using fluoroscopy. In some instances, disposed within the hydrophilic coating 904 on the proximal end 708 of the outer/cutting sheath 546 are a set of visible markers 908 to enable tracking and to identify a depth of the outer/cutting sheath 546 by the human operator. In some instances, a hemostasis valve 910 may disposed on the distal end of the sheath lumen 712. In that regard, the hemostasis valve 910 is designed to minimize fluid loss and backflow through the sheath lumen 712. In some instances, where the outer/cutting sheath 546 is a cutting sheath, one or more sharp edges 914 may be disposed at the distal end 710 of the outer/cutting sheath 546 forming cutting tip 720. In that regard, the sharp edges 914 may be a blade, a knife edge, a cutting edge, etc. The outer/cutting sheath 546 may be coupled to a motor (motor 544 of Fig. 5) that causes rotation of the sharp edges 914 such that such that the one or more sharp edges 914 cut and/or resect the degenerated leaflets of the natural aortic valve or the replacement aortic valve. Aspects of the outer/cutting sheath 546 may include features similar to the TightRail^{®} mechanical rotation dilator sheath available from Koninklijke Philips NV.

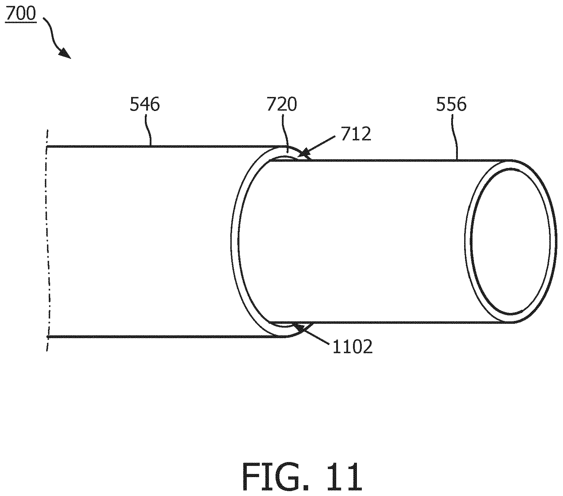

Fig. 11 is a diagrammatic view of an intraluminal device 700 according to aspects of the present disclosure. Visible are the inner sheath 556 disposed within the sheath lumen 712 of the outer/cutting sheath 546. In some instances, an outside diameter of the inner sheath 556 is smaller in diameter than an inside diameter of the outer/cutting sheath 546, thereby forming a circumferential gap 1102. In some instances, the circumferential gap 1102, which is the difference between the outside diameter of the inner sheath 556 and the inside diameter of the outer/cutting sheath 546, is 1-5 millimeters. In some instances, the circumferential gap 1102 is 2-5 millimeters. In some instances, the circumferential gap 1102 is 2-3 millimeters. In that regard, the circumferential gap 1102 is sufficient in size for leaflets, such as the aortic valve leaflets 304 of Fig. 4A, to be drawn into circumferential gap 1102 in order that a portion of the leaflets may be resected in anticipation of a new replacement TAVR valve. In some instances, a cutting tip 720 is disposed at the distal end of the outer/cutting sheath 546.

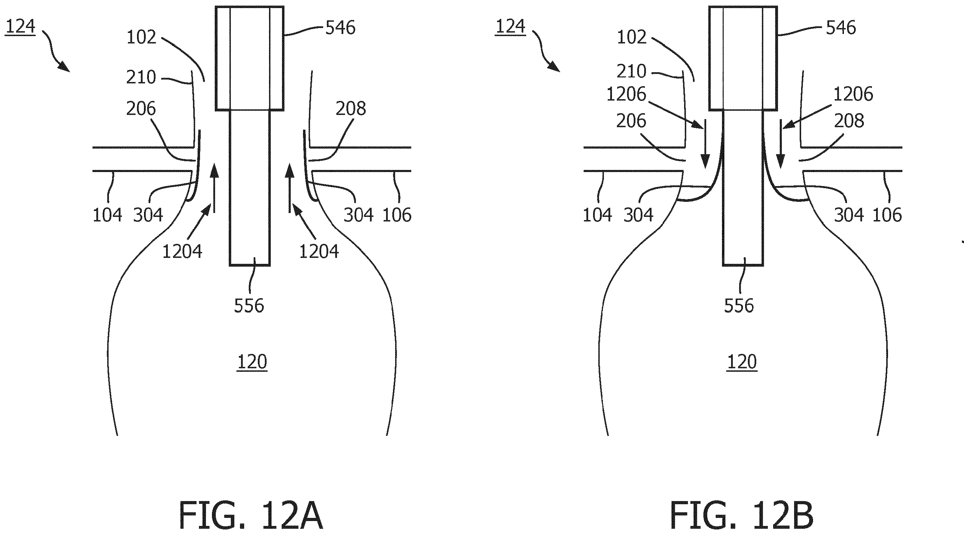

Figs. 12A and 12B are diagrammatic cross-sectional side views of an aortic valve 124 according to aspects of the present disclosure. Visible are the natural heart wall 210, the aortic valve leaflets 304, the right coronary artery 104, and the left main coronary artery 106. In the aspects of Fig. 12A, during systole indicated by directional arrows 1204, the aortic valve leaflets 304 open and allow blood to flow from the left ventricle 120 to the aorta 102 indicated by directional arrows 1204. In that regard, during systole, the inner sheath 556 is advanced between the aortic valve leaflets 304. In the aspects of Fig. 12B, during diastole indicated by directional arrows 1206, the aortic valve leaflets 304 may be pushed by the flow of blood from the aorta 102 toward the left ventricle 120 such that the aortic valve leaflets 304 rest against and/or contact the inner sheath 556 to prevent mitral regurgitation.

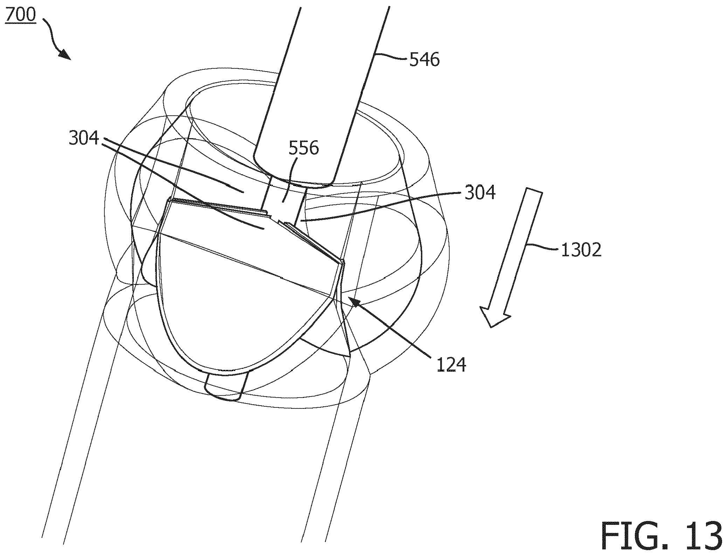

Fig. 13 is diagrammatic view of an intraluminal device 700 inserted into an aortic valve 124 of a human heart according to aspects of the present disclosure. Visible are the outer/cutting sheath 546 and the inner sheath 556. In the aspects of Fig. 13, the inner sheath 556 has been advanced through the aortic valve 124 between the aortic valve leaflets 304 as indicated by directional arrow 1302. In some instances, during diastole, the aortic valve leaflets 304 may be pushed by the flow of blood from the aorta toward the left ventricle such that the aortic valve leaflets 304 rest against the inner sheath 556.

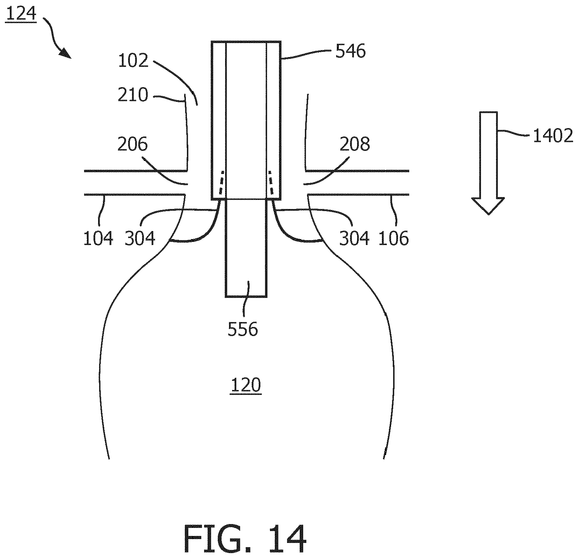

Fig. 14 is a diagrammatic cross-sectional side view of an aortic valve 124 according to aspects of the present disclosure. Visible are the natural heart wall 210, the aortic valve leaflets 304, the right coronary artery 104, and the left main coronary artery 106. In the aspects of Fig. 14, during diastole, the aortic valve leaflets 304 may be pushed by the flow of blood from the aorta 102 toward the left ventricle 120 such that the aortic valve leaflets 304 rest against the inner sheath 556. In that regard, during diastole, the outer/cutting sheath 546 is advanced distally towards the distal portion of the inner sheath 546 as indicated by directional arrow 1402 pinning the aortic valve leaflets 304 in the gap between the outer diameter of the inner sheath 556 and the inner diameter of the outer/cutting sheath 546.

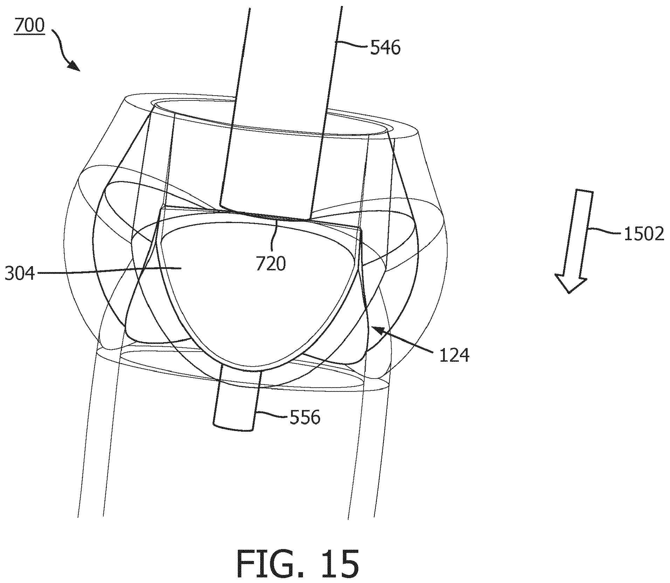

Fig. 15 is diagrammatic view of an intraluminal device 700 inserted into an aortic valve 124 of a human heart according to aspects of the present disclosure. Visible are the outer/cutting sheath 546 and the inner sheath 556. In the aspects of Fig. 15, the outer/cutting sheath 546 has been advanced, as indicated by directional arrow 1502, to capture the aortic valve leaflets 304 in the gap between the outer diameter of the inner sheath 556 and the inner diameter of the outer/cutting sheath 546. In that regard, the cutting tip 720 at the distal end of the outer/cutting sheath 546 operates to resect a portion of the aortic valve leaflets 304 as the outer/cutting sheath 546 advances through the aortic valve leaflets 304. In some instances, the aortic valve leaflets 304 may be captured via a suction from a pump, such as pump 552 of Fig. 5, between the inner sheath 556 and the outer/cutting sheath 546 such that the aortic valve leaflets 304 are suctioned into a gap between an outside of the inner sheath 556 and an inside of the outer/cutting sheath 546. In that regard, the suction may be for receiving the leaflet within the gap, capturing the cut portion of the leaflet within the gap, and/or removing the cut portion of the leaflet from the body. In some instances, the aortic valve leaflets 304 may be captured by Archimedes screw or helical retraction, based on a rotation of motor 554 of Fig. 5, the outside of the inner sheath 556 moves the aortic valve leaflets 304 between an outside of the inner sheath 556 and an inside of the outer/cutting sheath 546. In that regard, the screw thread and/or rotation may be for receiving the leaflet within the gap, capturing the cut portion of the leaflet within the gap, and/or removing the cut portion of the leaflet from the body. In some instances, the outside of the inner sheath 556 may comprise a frictional material, texturized, and/or coated with a friction surface such that, during diastole, the aortic valve leaflets 304 of the aortic valve 124 adhere to the outer surface of the inner sheath 556 and, as the outer/cutting sheath is advanced, positions the leaflets between an outside of the inner sheath 556 and an inside of the outer/cutting sheath 546. In that regard, the friction material may be for receiving the leaflet within the gap, capturing the cut portion of the leaflet within the gap, and/or removing the cut portion of the leaflet from the body. In some instances, movement of the inner sheath 556 alone (e.g., rotating the inner sheath 556 or translating the inner sheath 556), moves the cut portion of the leaflets along with the inner sheath 556. In some instances, movement of the inner sheath 556 and the outer/cutting sheath 546 moves the cut portion of the leaflets along with the inner sheath 556 and the outer/cutting sheath 546.

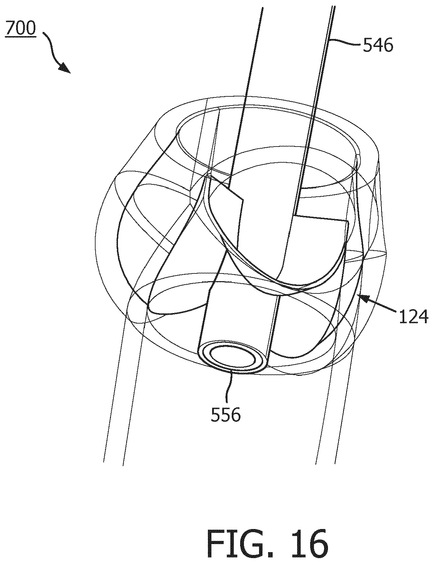

Fig. 16 is diagrammatic view of an intraluminal device 700 inserted into an aortic valve 124 of a human heart according to aspects of the present disclosure. Visible are the outer/cutting sheath 546 and the inner sheath 556. In the aspects of Fig. 16, the outer/cutting sheath 546 has been advanced through the aortic valve 124 resecting portions of the aortic valve leaflets 304.

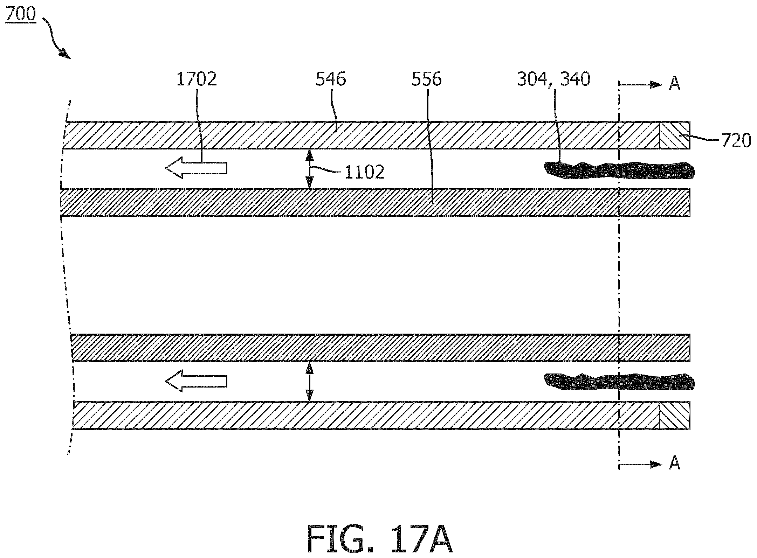

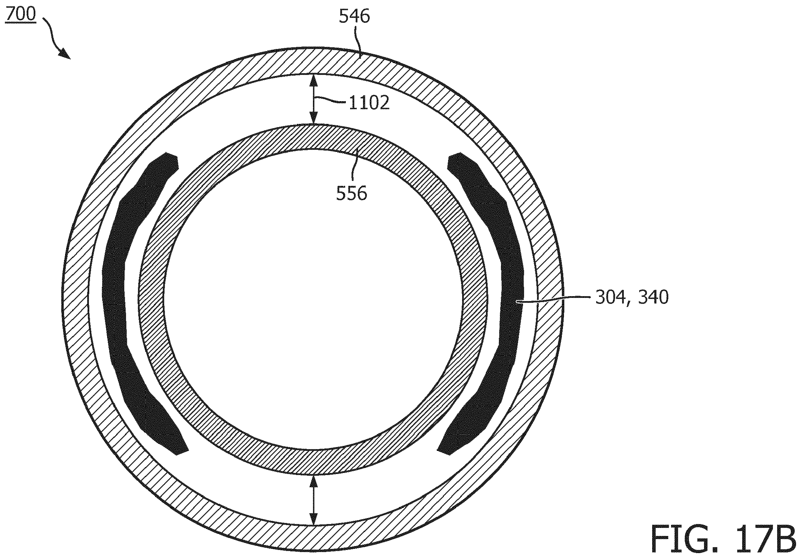

Fig. 17A is a diagrammatic cross-sectional top-view of an intraluminal device 700 according to aspects of the present disclosure. Visible are the outer/cutting sheath 546 and the inner sheath 556. In the aspects of Fig. 17A, a negative pressure and/or suction 1702 is applied via a pump, such as pump 552 of Fig. 5, which make the aortic valve leaflets 304 being introduced into the circumferential gap 1102 between an outside of the inner sheath 556 and an inside of the outer/cutting sheath 546, capturing the aortic valve leaflets 304 in the circumferential gap 1102. In some aspects, the suction 1702 pulls the aortic valve leaflets 304 into the gap 1102 prior to the leaflets 304 being resected. In some aspects, the suction 1702 maintains resected portions 340 of the aortic valve leaflets within the gap 1102 after to the leaflets have been resected. In some instances, a cutting tip 720 is disposed at the distal end of the outer/cutting sheath 546 to resect portions 340 of the aortic valve leaflets 304 once the aortic valve leaflets 304 are captured in the circumferential gap 1102 between an outside of the inner sheath 556 and an inside of the outer/cutting sheath 546. Fig. 17B illustrates a cross-sectional view of the intraluminal device 700 as seen along the lines of the section A-A of Fig. 17A taken therein, according to aspects of the present disclosure. Visible are the outer/cutting sheath 546 and the inner sheath 556. In the aspects of Fig. 17B, the aortic valve leaflets 304 and/or resected portions 340 are pulled into the circumferential gap 1102 between an outside of the inner sheath 556 and an inside of the outer/cutting sheath 546 capturing the aortic valve leaflets 304 and/or resected portions 340 in the circumferential gap 1102. In some instances, the suction 1702 moves the resected portions 340 through all or nearly all of the length of the gap, to the proximal portion of the outer/cutting sheath 546 and/or the inner sheath 556 that is outside of the body such that the resected portions 340 are removed from the body.

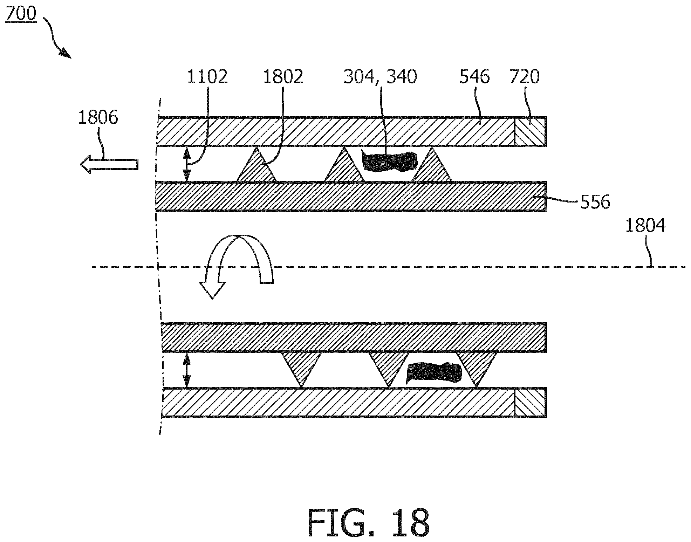

Fig. 18 is a diagrammatic cross-sectional top-view of an intraluminal device 700 according to aspects of the present disclosure. Visible are the outer/cutting sheath 546 and the inner sheath 556. In the aspects of Fig. 18, disposed on an outer surface of the inner sheath 556 is a helical retraction structure 1802 (e.g., Archimedes screw) that extends radially outward, which when rotated by a motor, such as motor 554 of Fig. 5, about a longitudinal axis 1804 drags and/or pulls the aortic valve leaflets 304 into the circumferential gap 1102 between an outside of the inner sheath 556 and an inside of the outer/cutting sheath 546 in a direction as indicated by directional arrow 1806. In some aspects, the helical retraction structure 1802 pulls the aortic valve leaflets 304 into the gap 1102 prior to the leaflets 304 being resected. In some aspects, the helical retraction structure 1802 maintains resected portions 340 of the aortic valve leaflets within the gap 1102 after to the leaflets have been resected. In some instances, a cutting tip 720 is disposed at the distal end of the outer/cutting sheath 546 to resect portions of the aortic valve leaflets 304 once the aortic valve leaflets 304 are captured in the circumferential gap 1102 between an outside of the inner sheath 556 and an inside of the outer/cutting sheath 546. In some instances, the helical retraction structure 1802 moves the resected portions 340 through all or nearly all of the length of the gap, to the proximal portion of the outer/cutting sheath 546 and/or the inner sheath 556 that is outside of the body such that the resected portions 340 are removed from the body.

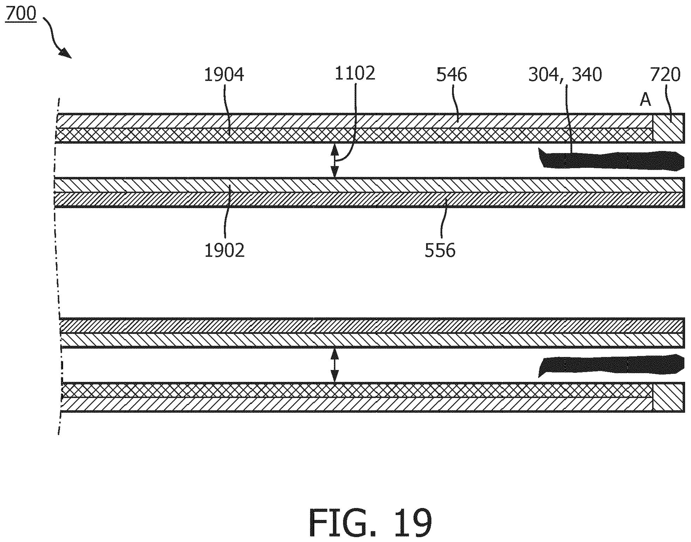

Fig. 19 is a diagrammatic cross-sectional top-view of an intraluminal device 700 according to aspects of the present disclosure. Visible are the outer/cutting sheath 546 and the inner sheath 556. In the aspects of Fig. 19, disposed on an outer surface of the inner sheath 556 may be a feature 1902. In some aspects, the feature 1902 may be a frictional material. In some aspects, the feature 1902 may be a texture. In some aspects, the feature 1902 may be a coating. In that regard, the feature 1902 may attract and/or adhere to aortic valve leaflets 304, causing the aortic valve leaflets to contact the frictional surface, and drawing the aortic valve leaflets 304 into the circumferential gap 1102 between an outside of the inner sheath 556 and an inside of the outer/cutting sheath 546. In the aspects of Fig. 19, disposed on an inner surface of the outer/cutting sheath 546 may be a feature 1904. In some aspects, the feature 1904 may be a frictional material. In some aspects, the feature 1904 may be a texture. In some aspects, the feature 1904 may be a coating. In that regard, the feature 1904 may attract and/or adhere to aortic valve leaflets 304 drawing them into the circumferential gap 1102 between an outside of the inner sheath 556 and an inside of the outer/cutting sheath 546. In some aspects, only one of the feature 1902 or feature 1904 may be provided. In some aspects, both feature 1902 and feature 1904 may be provided. Features 1902 and/or 1904 can include etching or grafting on the sheath surfaces to increase friction such as lattice patterns, slight frictional material, or hydrophobic coatings. These could include PTFE-based coatings, silicone like materials or materials with impregnated particles. In some aspects, when both feature 1902 and feature 1904 are provided, both feature 1902 and feature 1904 are a same feature. In some aspects, when both feature 1902 and feature 1904 are provided, feature 1902 may be a different feature that the feature 1904. In some aspects, the feature 1902 and/or the feature 1904 pulls the aortic valve leaflets 304 into the gap 1102 prior to the leaflets 304 being resected. In some aspects, the feature 1902 and/or the feature 1904 maintains resected portions 340 of the aortic valve leaflets within the gap 1102 after to the leaflets have been resected. In some instances, the feature 1902 and/or the feature 1904 facilitates movement of the resected portions 340 through all or nearly all of the length of the gap, to the proximal portion of the outer/cutting sheath 546 and/or the inner sheath 556 that is outside of the body such that the resected portions 340 are removed from the body. For example, the outer/cutting sheath 546 and/or the inner sheath 556 can be removed from the patient body while the feature 1902 and/or the feature 1904 holds the resected portions 340.

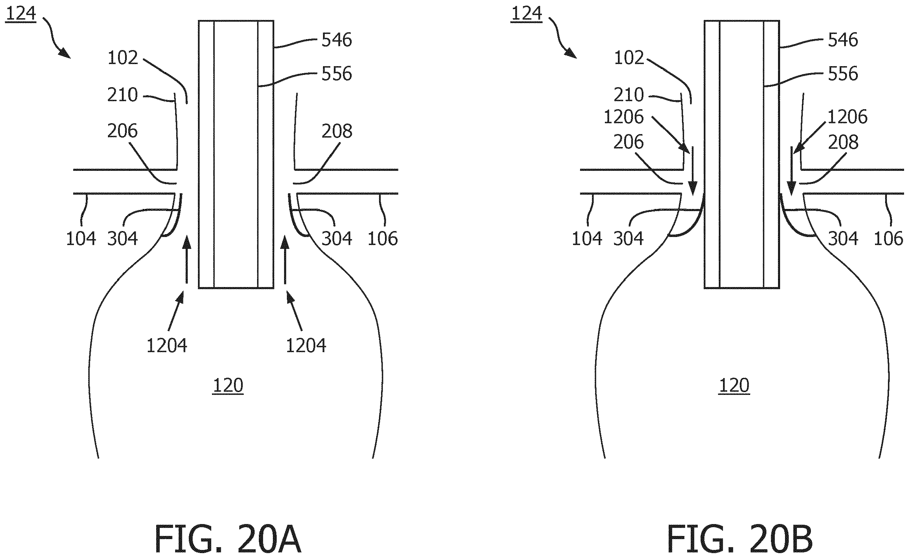

Figs. 20A and 20B are diagrammatic cross-sectional side views of an aortic valve 124 according to aspects of the present disclosure. Visible are the natural heart wall 210, the aortic valve leaflets 304, the right coronary artery 104, and the left main coronary artery 106. In the aspects of Fig. 20A, after the aortic valve leaflets 304 have been resected by the advancement of the outer/cutting sheath 546 over the inner sheath 556 utilizing the cutting tip disposed on the distal end of the outer/cutting sheath 546, during systole indicated by directional arrows 1204, the aortic valve leaflets 304 open and allow blood to flow from the left ventricle 120 to the aorta 102 indicated by directional arrows 1204. It that regard, the resected aortic valve leaflets 304 do not obstruct the openings 206 and 208 to the right coronary artery 104 and the left main coronary artery 106, respectively, allowing the oxygenated blood to flow to the muscles of the heat via the right coronary artery 104, and the left main coronary artery 106. In the aspects of Fig. 20B, during diastole indicated by directional arrows 1206, the aortic valve leaflets 304 may be pushed by the flow of blood from the aorta 102 toward the left ventricle 120 such that the aortic valve leaflets 304 rest against the outer/cutting sheath 546.

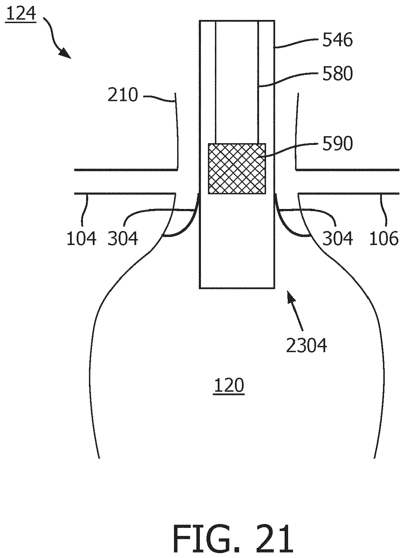

Fig. 21 is a diagrammatic cross-sectional side view of an aortic valve 124 according to aspects of the present disclosure. Visible are the natural heart wall 210, the aortic valve leaflets 304, the right coronary artery 104, and the left main coronary artery 106. In some aspects, after the aortic valve leaflets 304 have been resected by the advancement of the outer/cutting sheath 546 over the inner sheath 556, the inner sheath 556 is withdrawn from the outer/cutting sheath 546 while the outer/cutting sheath 546 remains in place so that the lumen of the outer/cutting sheath 546 is open. In some aspects, withdrawing the inner sheath 556 takes the resected portions 340 of the aortic valve leaflets 304 along with it. In other aspects, the resected portions 340 can be removed without removing the inner sheath 556 (e.g., suction 1702 and/or helical retraction 1802). The inner sheath 556 can be removed so that the lumen of the outer/cutting sheath 546 is open. In some aspects, the TAVR delivery catheter 580, along with the TAVR valve 590 disposed at the distal end of the TAVR delivery catheter 580, is inserted into the outer/cutting sheath 546, positioned within the body lumen, and advanced to the aortic valve 124 region.

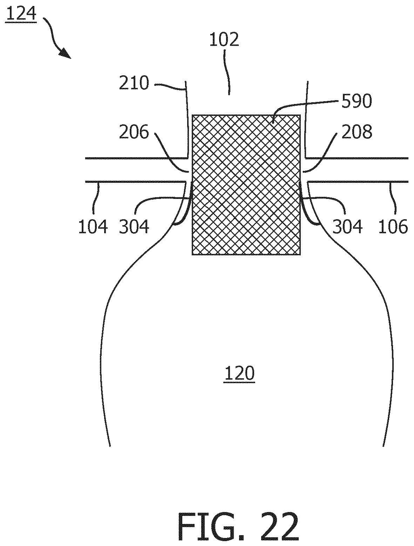

Fig. 22 is a diagrammatic cross-sectional side view of an aortic valve 124 according to aspects of the present disclosure. Visible are the natural heart wall 210, the aortic valve leaflets 304, the right coronary artery 104, and the left main coronary artery 106. In some aspects, the TAVR valve 590 is moved into position between the aorta 102 and the left ventricle 120. In some aspects, once positioned, the TAVR valve 590 is expanded pressing the resected aortic valve leaflets 304 against the natural heart wall 210. In that regard, the resected aortic valve leaflets 304 fail to obstruct the openings 206 and 208 to the right coronary artery 104 and the left main coronary artery 106, respectively.

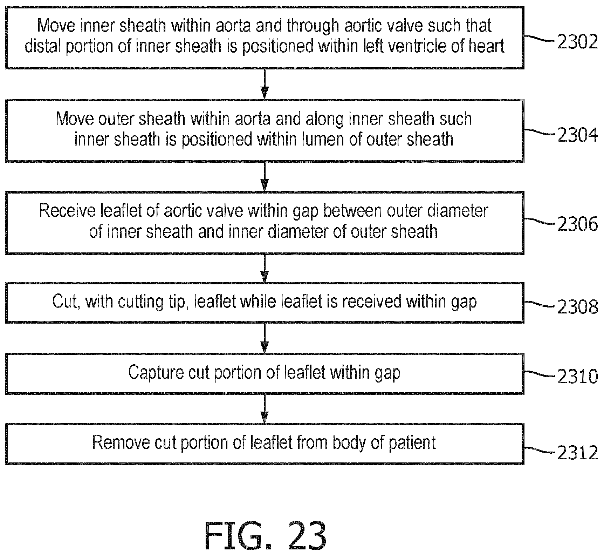

Fig. 23 is a flow diagram of the process performed in removing one or more portions of leaflets of a degenerated natural aortic valve or a degenerated replacement aortic valve according to aspects of the present disclosure. In step 2302, an inner sheath is moved within an aorta and through an aortic valve such that a distal portion of the inner sheath is positioned within a left ventricle of a heart. Aspects of step 2302 are illustrated and described with respect to Figs. 12A, 12B, and 13. In step 2304, an outer sheath is moved within the aorta and along the inner sheath such the inner sheath is positioned within a lumen of the outer sheath. The outer sheath comprises a distal portion and a cutting tip positioned at the distal portion of the outer sheath. Aspects of step 2304 are illustrated and described with respect to Figs. 14 and 15. In step 2306, a leaflet of the aortic valve is received within a gap between an outer diameter of the inner sheath and an inner diameter of the outer sheath. Aspects of step 2306 are illustrated and described with respect to Figs. 14 and 15. In step 2308, the leaflet is cut, with the cutting tip, while the leaflet is received within the gap. Aspects of step 2308 are illustrated and described with respect to Fig. 16. In step 2310, the cut portion of the leaflet is captured within the gap. Aspects of step 2310 are illustrated and described with respect to Figs. 17A, 17B, 18, and 19. In step 2312, the cut portion of the leaflet is removed from a body of a patient. Aspects of step 2312 are illustrated and described with respect to Fig. 21.

Accordingly, it may be seen that the disclosed apparatus advantageously enables the replacement of an aortic valve in a non-obstructive way by removing old valve leaflets and preparing the implant site for a cleaner valve deployment and operation. By resection portions of the leaflets of the degenerated aortic valve, the replacement valve may be expanded without the current leaflets covering the openings to the coronary arteries, thereby allowing oxygenated blood to flow to the muscles of the heart.

The logical operations making up the aspects of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use electrical and electronic devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of aspects of the present disclosure. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the present disclosure, e.g., as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. A system, comprising:
an outer sheath configured to be positioned within a heart of a patient, wherein the outer sheath comprises a proximal portion, a distal portion, a lumen, a cutting tip positioned at the distal portion; and
an inner sheath configured to be positioned within the lumen of the outer sheath, wherein the inner sheath comprises a proximal portion and a distal portion;
wherein the outer sheath and the inner sheath are configured to receive a leaflet of a valve within a gap between an outer portion of the inner sheath and an inner portion of the outer sheath,
wherein the cutting tip of the outer sheath is configured to cut the leaflet while the leaflet is received within the gap such that a cut portion of the leaflet is captured within the gap for removal from the patient.

2. The system of claim 1, wherein an outer surface of the inner sheath and/or an outer surface of the outer sheath comprises a hydrophilic coating.

3. The system of claim 1 or 2,
wherein at least one of the proximal portion of the inner sheath and the proximal portion of the outer sheath comprises a hemostasis valve.

4. The system of any of the preceding claims,
wherein at least one of the distal portion of the inner sheath and the distal portion of the outer sheath comprises a radiopaque marker.

5. The system of any of the preceding claims,
wherein at least one of the proximal portion of the inner sheath and the proximal portion of the outer sheath comprises a visible marker.

6. The system of any of the preceding claims, wherein the cutting tip comprises at least one sharp edge and/or at least one optical fiber.

7. The system of any of the preceding claims, further comprising a pump configured to provide suction within the gap such that the cut portion of the leaflet is captured within the gap.

8. The system of any of the preceding claims,
wherein an outer surface of the inner sheath comprises a screw thread.

9. The system of claim 8,
wherein the inner sheath is configured to be rotated relative to the outer sheath such that the cut portion of the leaflet is captured within the gap by the screw thread.

10. The system of claim 9, further comprising a motor configured to rotate the inner sheath.

11. The system of any of the claims 1 to 7,
wherein at least one of an inner surface of the outer sheath and an outer surface of the inner sheath comprises a frictional material configured to capture the cut portion of the leaflet within the gap.

12. The system of any of the claims 1 to 7 and 11,
wherein at least one of an inner surface of the outer sheath and an outer surface of the inner sheath comprises a texture.

13. The system of any of the preceding claims, further comprising:
a valve delivery catheter configured to be positioned within the lumen of the outer sheath,
wherein the valve delivery catheter is movable relative to the outer sheath.

14. The system of claim 13,
wherein the valve delivery catheter comprises a transcatheter aortic valve replacement valve removably coupled at a distal portion of the valve delivery catheter.

15. A method, comprising:
moving an inner sheath within an aorta and through an aortic valve such that a distal portion of the inner sheath is positioned within a left ventricle of a heart;
moving an outer sheath within the aorta and along the inner sheath such the inner sheath is positioned within a lumen of the outer sheath, wherein the outer sheath comprises a distal portion and a cutting tip positioned at the distal portion of the outer sheath;
receiving a leaflet of the aortic valve within a gap between an outer portion of the inner sheath and an inner portion of the outer sheath;
cutting, with the cutting tip, the leaflet while the leaflet is received within the gap;
capturing a cut portion of the leaflet within the gap; and
removing the cut portion of the leaflet from a body of a patient.
